# EUROPEAN PATENT SPECIFICATION

(11) **EP 2 409 689 B1**
(45) Date of publication and mention of the grant of the patent: **23.04.2014**
(21) Application number: 11174408.2
(22) Date of filing: 18.07.2011
(51) Int. Cl.: A61K 9/20, A61K 31/4365, A61P 7/02, A61P 9/00, A61K 9/50

(54) **Prasugrel tablet formulations**
Prasugrel-Tablettenformulierungen
Formulations de comprimés de prasugrel

(30) Priority: 29.09.2010 TR 201007926; 17.08.2010 TR 201006802; 19.07.2010 TR 201005900
(43) Date of publication of application: 25.01.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Öner, Levent, 34398 Istanbul (TR)
(74) Representative: Sevinç, Erkan

(56) References cited:
- EP-A1- 2 100 607
- WO-A2-2008/073759
- US-A1- 2009 281 136
- US-A1- 2009 291 138
- DATABASE WPI Week 200379 Thomson Scientific, London, GB; AN 2003-847929 XP002619601, -& JP 2003 246735 A (DAIICHI SEIYAKU CO LTD) 2 September 2003 (2003-09-02)

## Description

### Field of Invention

The present invention relates to formulations of prasugrel or a pharmaceutically acceptable salt of prasugrel. The present invention particularly relates to stable tablet formulations of prasugrel with desired levels of solubility and dissolution rate.

### Background of Invention

Prasugrel hydrochloride is a member of the thienopyridine class and inhibits the activation and aggregation of platelets by means of P2Y12 and ADP receptors. Its chemical designation is 2-acetoxy-5-(α-cyclopropylcarbonyl-2-fluorobenzyl)-4,5,6,7-tetrahydrothieno [3,2-c]pyridine hydrochloride, with the chemical structure illustrated below in Formula 1.

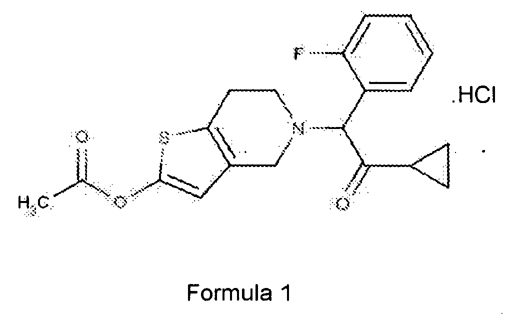

Prasugrel hydrochloride is a white solid. Prasugrel hydrochloride is well soluble at pH 2, weakly soluble at pH 3 to 4, and is substantially insoluble at pH 6 to 7.5.

Prasugrel hydrochloride is marketed under the trademark Effient® in 5 or 10 mg dosages. It is initially administered in a dosage amount of 60 mg, as a loading dose. It is used in preventing or treating thrombosis and cardiovascular diseases.

Searching the patent literature reveals various patents in relation to prasugrel.

Patent application EP1298132, for instance, discloses a prasugrel hydrochloride salt, a method for obtaining this salt, and its use for thrombosis and embolism.

Patent application EP1728794 discloses a prasugrel maleate salt, a method for obtaining this salt, and its use for thrombosis and embolism.

JP 2003246735 A discloses formulations of prasugrel that can be in the form of a tablet, a capsule, a granule, a powder, or a syrup formulation etc. Pullulan is mentioned as filler.

US 2009291138 A1 discloses film coated preparations comprising prasugrel or prasugrel HCl and a film layer which is coated on the formulation, said film layer having one or more film coating base agents selected from polyvinyl alcohol, sodium carboxymethyl cellulose and pullulan. The formulation has improved stability.

EP 2100607 A1 discloses a pharmaceutical composition comprising prasugrel and a water soluble polymer, selected from hydroxypropylmethyl cellulose, hydroxypropyl cellulose or polyvinylpyrrolidone which has improved stability. The pharmaceutical composition is preferably in a solid dosage form, for example, tablets, capsules, granules, fine granules, powders, pills, chewables or troches. Among the excipients, pullulan is suggested as a filler or coating agent.

US 2009281136 A1 discloses stable pharmaceutical formulations comprising prasugrel or a pharmaceutically acceptable salt thereof; at least one antioxidant; and one or more pharmaceutically acceptable excipients. The use of package materials is also disclosed.

WO 2008073759 A2 discloses manufacture of tablets, caplets, capsules or other solid form of prasugrel, which involves placing tablets, caplets, capsules or other solid form of prasugrel in an air and/or moisture impervious bottle, optionally adding a desiccant and an oxygen scavenger, adding liquid nitrogen and sealing the air and/or moisture impervious bottle under a positive liquid nitrogen pressure. The examples disclose prasugrel tablets which are film coated with HPMC.

Patent application WO2006135605 discloses a formulation containing prasugrel hydrochloride, packaged in an air and moisture impervious gas-inerted blister pack.

Stability-related problems do occur in a plurality of active agents, including prasugrel, under the influence of ambient and physical conditions. Prasugrel is an active agent that is highly-susceptible to air and humidity. When prasugrel is exposed to air and humidity, it degrades structurally and develops behavioral changes. As a result of this fact, two main problems emerge. The first problem is that the stability of prasugrel products developed is not of desired level and the shelf life thereof is shortened. The second problem is that prasugrel is reactive against the excipients employed in developing formulations containing prasugrel. This fact causes impurities to occur in formulation and leads to incorporation of undesired components into the formulation.

Due to the abovementioned drawbacks, a novelty is required in the art of prasugrel hydrochloride formulations used for preventing and treating thrombosis and cardiovascular diseases.

### Object and Brief Description of Invention

The present invention provides a prasugrel formulation, eliminating all aforesaid problems and brining additional advantages to the relevant prior art.

Accordingly, the main object of the present invention is to obtain a formulation of prasugrel, which is stable and has desired levels of solubility and dissolution rate.

Another object of the present invention is to develop a multi-coating, providing the stability of prasugrel-containing formulation and not affecting the solubility and dissolution rates thereof.

A further object of the present invention is to obtain a stable prasugrel formulation with high bioavailability.

Another object of the present invention is to provide a high dissolution rate for such stable prasugrel formulations with high bioavailability.

A further object of the present invention is to avoid any aggregation of ingredients of the composition and to provide a desired level of flowability during production, thanks to convenient excipients used while the formulation is obtained.

A solid pharmaceutical tablet formulation has been developed to carry out all objects, referred to above and to emerge from the following detailed description.

According to a preferred embodiment of the present invention, said novelty comprises prasugrel, or a pharmaceutically acceptable salt thereof and pullulan as a filling agent.

The weight ratio of prasugrel to pullulan is between 0.25 to 10, and preferably 0.25 to 4.

According to a preferred embodiment of the present invention, prasugrel or a pharmaceutically acceptable salt thereof is used in a granular form.

According to another preferred embodiment of the present invention, said granules of prasugrel, or of a pharmaceutically acceptable salt thereof, further comprise at least one coating layer which contains pullulan.

Another preferred embodiment according to the present invention further comprises at least one or a proper mixture of binders, lubricants, and glidants.

According to a preferred embodiment of the present invention, said binder is at least one or a mixture of polyvinylpyrrolidone (povidone), hydroxypropyl methylcellulose, hydroxypropyl cellulose, carboxymethyl cellulose, methyl cellulose, ethyl cellulose, and other cellulose derivatives, and gelatin, with polyvinylpyrrolidone being preferred.

According to a preferred embodiment of the present invention, said glidants comprise at least one or a mixture of colloidal silicone dioxide, talk, aluminum silicate, magnesium silicate, with colloidal silicone dioxide being preferred.

According to a preferred embodiment of the present invention, suitable disintegrants include at least one or a mixture of sodium starch glycolate, croscarmellose sodium, crospovidone, sodium alginate, gums, starch, and magnesium aluminum silicate, with croscarmellose sodium being preferred.

According to a preferred embodiment of the present invention, suitable lubricants include at least one or a mixture of sodium stearyl fumarate, magnesium stearate, polyethylene glycol, stearic acid, metal stearates, boric acid, sodium chloride benzoate and acetate, sodium or magnesium lauryl sulfate, with magnesium stearate being preferred.

A preferred embodiment according to the present invention comprises at least one coating layer. The coating layer comprises at least one or a mixture of polyvinyl alcohol, methacrylic acid derivatives, hydroxypropyl methylcellulose, and carboxymethyl cellulose.

A further preferred embodiment according to the present invention provides a method for preparing a solid pharmaceutical formulation, this method comprising the steps of
a. coating the particles of the active agent with pullulan in a fluidized bed system,
b. mixing the pullulan-coated active agent particles with a filler, binder, disintegrant, and lubricant-glidants,
c. compressing the mixture into tablets, and
d. film-coating the tablets compressed.

In a further preferred embodiment according to the present invention, said pharmaceutical formulation comprises the following ingredients:
a. core
   a. prasugrel or a pharmaceutically acceptable salt or polymorph thereof at 1 to 20% by weight,
   b. pullulan at 0.1 to 4% by weight,
   c. microcrystalline cellulose at 5.0 to 90% by weight,
   d. polyvinylpyrrolidone at 0.25 to 5% by weight,
   e. croscarmellose sodium at 0.2 to 10% by weight,
   f. colloidal silicone dioxide at 0.1 to 5% by weight,
   g. magnesium stearate at 0.1 to 10% by weight;
b. coating
   a. polyvinyl alcohol at 0.5 to 5% by weight, or
   b. hydroxypropyl methylcellulose at 0.5 to 5% by weight, or a properly-proportioned mixture thereof.

### Detailed Description of Invention

### Example

| **Unit Formula** | **Amount in tablet (%)- Example 1** | **Amount in tablet (%)- Example 2** | **Amount in tablet (%)- Example 3** |
|---|---|---|---|
| Core tablet | | | |
| prasugrel | 5 | 5 | 5 |
| pullulan | - | 1.25 | 3.5 |
| microcrystalline cellulose | 80.5 | 79.25 | 77 |
| polyvinylpyrrolidone | 4 | 4 | 4 |
| croscarmellose sodium | 4 | 4 | 4 |
| colloidal silicone dioxide | 0.5 | 0.5 | 0.5 |
| magnesium stearate | 1 | 1 | 1 |

| **Film coating** | | | |
|---|---|---|---|
| Opadry AMB -OY- B 28920 white | 5 | 5 | 5 |
| Prasugrel/pullulan | - | 4 | 1.42 |

The formulation according to the present invention is prepared as follows. The active agent particles are coated with pullulan in a fluidized bed system. Then, pullulan-coated active agent particles are mixed with pullulan, microcrystalline cellulose, polyvinylpyrrolidone, croscarmellose sodium, colloidal silicone dioxide, and magnesium stearate. The mixture is compressed into tablets. Finally, the tablets compressed are coated with a film comprising Opadry AMB -OY- B 28920 white or Opadry II 85 F28751 white, or a properly-proportioned mixture thereof. Opadry AMB -OY- B 28920 white comprises polyvinyl alcohol, whereas Opadry II 85 F28751 white comprises hydroxypropyl methylcellulose. Said tablets are packaged into alu-alu blister.

The term granule, as used herein, means a powder, particle, or a pellet form of prasugrel or of a pharmaceutically acceptable salt of prasugrel.

### Stability Test Results

| **Samples** | **Remaining 4 weeks at 55 C° and % 85 relative humidity** |
|---|---|
| Amount of active ingredient of Example 1 | % 89 |
| Amount of active ingredient of Example 2 | % 92 |
| Amount of active ingredient of Example 3 | % 96 |

With the invention, it has been surprisingly found that stable prasugrel formulations can be obtained which show good solubility and dissolution rates, and thus high bioavailability.

The formulation obtained is used in preventing or treating thrombosis, embolism and cardiovascular diseases.

It is further possible to use the following additional excipients in the formulation.

Suitable coating agents include, but are not restricted to, hydroxypropyl methylcellulose, polyethylene glycol, polyvinylpyrrolidone, polyvinylpyrrolidone-vinyl acetate copolymer (PVP-VA), pullulan like polymers, and all kinds of Opadry, as well as pigments, dyes, titanium dioxide and iron oxide, talk.

Suitable colorants include, but are not restricted to, at least one or a mixture of food, drug, and cosmetic (FD&C) dyes (FD&C blue, FD&C green, FD&C red, FD&C yellow, FD&C lake), ponceau, indigo drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

Suitable preservatives include, but are not restricted to, at least one or a mixture of methylparaben and propylparaben and salts thereof (e.g. sodium or potassium salts), sodium benzoate, citric acid, benzoic acid, butylated hydroxytoluene and butylated hydroxyanisole, etc..

The protection scope of the present invention is set forth in the annexed claims and cannot be restricted to the illustrative disclosures given above, under the detailed description.

## Claims

1. A solid pharmaceutical tablet formulation, comprising prasugrel, or a pharmaceutically acceptable salt thereof and pullulan as a filling agent **characterized in that** the weight ratio of prasugrel to pullulan is between 0.25 to 10.

2. The solid pharmaceutical tablet formulation according to claim 1, wherein prasugrel, or a pharmaceutically acceptable salt thereof, is in a granular form.

3. The solid pharmaceutical tablet formulation according to any of the preceding claims, wherein the granules of prasugrel, or of a pharmaceutically acceptable salt thereof, further comprise at least one coating layer which contains pullulan.

4. The solid pharmaceutical tablet formulation according to any of the preceding claims, further comprising at least one or a mixture of binders, lubricants, and glidants.

5. The solid pharmaceutical tablet formulation according to any of the preceding claims, wherein said binder is selected from the group consisting of polyvinylpyrrolidone (povidone), hydroxypropyl methylcellulose, hydroxypropyl cellulose, carboxymethyl cellulose, methyl cellulose, ethyl cellulose, and gelatin.

6. The solid pharmaceutical tablet formulation according to any of the preceding claims, wherein said glidants is selected from the group consisting of colloidal silicone dioxide, talk, aluminum silicate, and magnesium silicate.

7. The solid pharmaceutical tablet formulation according to any of the preceding claims, wherein suitable disintegrant is selected from the group consisting of sodium starch glycolate, croscarmellose sodium, crospovidone, sodium alginate, gums, starch, and magnesium aluminum silicate.

8. The solid pharmaceutical tablet formulation according to any of the preceding claims, wherein said lubricant is selected from the group consisting of sodium stearyl fumarate, magnesium stearate, polyethylene glycol, stearic acid, metal stearates, boric acid, sodium or magnesium lauryl sulfate.

9. The solid pharmaceutical tablet formulation according to any of the preceding claims, comprising at least one coating layer.

10. The solid pharmaceutical tablet formulation according to any of the preceding claims, wherein said coating layer comprises at least one or a mixture of polyvinyl alcohol, methacrylic acid, hydroxypropyl methylcellulose, and carboxymethyl cellulose.

11. The solid pharmaceutical tablet formulation according to any of the preceding claims wherein said tablets are in an alu-alu blister.

12. The solid pharmaceutical tablet formulation according to any of the preceding claims, consisting of the following ingredients only:
a. core
i. prasugrel, or a pharmaceutically acceptable salt or polymorph thereof, at 1 to 20% by weight,
ii. pullulan at 0.2 to 4% by weight,
iii. microcrystalline cellulose at 5.0 to 90% by weight,
iv. polyvinylpyrrolidone at 0.25 to 5% by weight,
v. croscarmellose sodium at 0.2 to 10% by weight,
vi. colloidal silicone dioxide at 0.1 to 5% by weight,
vii. magnesium stearate at 0.1 to 10% by weight;
b. coating
i. polyvinyl alcohol at 0.5 to 5% by weight, or
ii. hydroxypropyl methylcellulose at 0.5 to 5% by weight, or a properly-proportioned mixture thereof.

13. The solid pharmaceutical tablet formulation according to any of the preceding claims for use in preventing or treating thrombosis, embolism, and cardiovascular diseases in mammalians, but particularly in humans.

14. A method for preparing a solid pharmaceutical tablet formulation according to any of the preceding claims, comprising the steps of
a. coating the particles of the active agent with pullulan in a fluidized bed system,
b. mixing the pullulan-coated active agent particles with a filler, binder, disintegrant, and lubricant-glidants,
c. compressing the mixture into tablets, and
d. film-coating the tablets compressed.

## Patentansprüche

1. Feste pharmazeutische Tablettenformulierung, umfassend Prasugrel oder ein pharmazeutisch verträgliches Salz davon und Pullulan als Füllmittel, **dadurch gekennzeichnet, dass** das Gewichtsverhältnis von Prasugrel zu Pullulan zwischen 0,25 und 10 liegt.

2. Feste pharmazeutische Tablettenformulierung nach Anspruch 1, wobei Prasugrel oder ein pharmazeutisch verträgliches Salz davon in granularer Form vorliegt.

3. Feste pharmazeutische Tablettenformulierung nach einem beliebigen der vorhergehenden Ansprüche, wobei das Granulat von Prasugrel oder eines pharmazeutisch verträglichen Salzes davon des Weiteren mindestens eine Beschichtungsschicht, die Pullulan enthält, umfasst.

4. Feste pharmazeutische Tablettenformulierung nach einem beliebigen der vorhergehenden Ansprüche, des Weiteren umfassend mindestens eine Mischung von Bindemitteln, Schmiermitteln und Gleitmitteln.

5. Feste pharmazeutische Tablettenformulierung nach einem beliebigen der vorhergehenden Ansprüche, wobei das Bindemittel ausgewählt ist aus der Gruppe bestehend aus Polyvinylpyrrolidon (Povidon), Hydroxypropylmethylcellulose, Hydroxypropylcellulose, Carboxymethylcellulose, Methylcellulose, Ethylcellulose und Gelatine.

6. Feste pharmazeutische Tablettenformulierung nach einem beliebigen der vorhergehenden Ansprüche, wobei das Gleitmittel ausgewählt ist aus der Gruppe bestehend aus kolloidem Siliziumdioxid, Talk, Aluminiumsilikat und Magnesiumsilikat.

7. Feste pharmazeutische Tablettenformulierung nach einem beliebigen der vorhergehenden Ansprüche, wobei ein geeignetes Sprengmittel ausgewählt ist aus der Gruppe bestehend aus Natriumstärkeglykolat, Croscarmelosenatrium, Crospovidon, Natriumalginat, Gummis, Stärke, und Magnesiumaluminiumsilikat.

8. Feste pharmazeutische Tablettenformulierung nach einem beliebigen der vorhergehenden Ansprüche, wobei das Schmiermittel ausgewählt ist aus der Gruppe bestehend aus Natriumstearylfumarat, Magnesiumstearat, Polyethylenglykol, Stearinsäure, Metallstearaten, Borsäure, Natrium oder Magnesiumlaurylsulfat.

9. Feste pharmazeutische Tablettenformulierung nach einem beliebigen der vorhergehenden Ansprüche, umfassend mindestens eine Beschichtungsschicht.

10. Feste pharmazeutische Tablettenformulierung nach einem beliebigen der vorhergehenden Ansprüche, wobei die Beschichtungsschicht mindestens eines oder eine Mischung von Polyvinylalkohol, Methacrylsäure, Hydroxypropylmethylcellulose, und Carboxymethylcellulose umfasst.

11. Feste pharmazeutische Tablettenformulierung nach einem beliebigen der vorhergehenden Ansprüche, wobei die Tabletten in einem Alu-Alu-Blister vorliegen.

12. Feste pharmazeutische Tablettenformulierung nach einem beliebigen der vorhergehenden Ansprüche, nur bestehend aus den folgenden Inhaltsstoffen:
a. Kern
i. Prasugrel oder ein pharmazeutisch verträgliches Salz oder Polymorph davon, bei 1 bis 20 Gewichts-%,
ii. Pullulan bei 0,2 bis 4 Gewichts-%,
iii. mikrokristalline Cellulose bei 5,0 bis 90 Gewichts-%,
iv. Polyvinylpyrrolidon bei 0,25 bis 5 Gewichts-%,
v. Croscarmellosenatrium bei 0,2 bis 10 Gewichts-%,
vi. kolloides Siliziumdioxid bei 0,1 bis 5 Gewichts-%,
vii. Magnesiumstearat bei 0,1 bis 10 Gewichts-%;
b. Beschichtung
i. Polyvinylalkohol bei 0,5 bis 5 Gewichts-%, oder
ii. Hydroxypropylmethylcellulose bei 0,5 bis 5 Gewichts-%, oder eine passend proportionierte Mischung davon.

13. Feste pharmazeutische Tablettenformulierung nach einem beliebigen der vorhergehenden Ansprüche, zur Verwendung in der Vorbeugung oder Behandlung von Thrombose, Embolie, und kardiovaskulären Krankheiten in Säugetieren, und insbesondere in Menschen.

14. Verfahren zum Herstellen einer festen pharmazeutischen Tablettenformulierung gemäß einem beliebigen der vorhergehenden Ansprüche, umfassend die Schritte von
a. Beschichten der Partikel des aktiven Mittels mit Pullulan in einem Wirbelschichtsystem,
b. Mischen der Pullulan-beschichteten Partikel des aktiven Mittels mit einem Füllstoff, Bindemittel, Sprengmittel, und Schmier-Gleitmitteln,
c. Komprimieren der Mischung in Tabletten, und
d. Film-beschichten der komprimierten Tabletten.

## Revendications

1. Formulation de comprimé pharmaceutique solide, comprenant du prasugrel, ou un sel pharmaceutiquement acceptable de celui-ci et du pullulane en tant qu'agent de remplissage **caractérisée en ce que** le rapport pondéral entre le prasugrel et le pullulane se situe entre 0,25 et 10.

2. Formulation de comprimé pharmaceutique solide selon la revendication 1, dans laquelle le prasugrel, ou un sel pharmaceutiquement acceptable de celui-ci, se présente sous une forme de granulés.

3. Formulation de comprimé pharmaceutique solide selon l'une quelconque des revendications précédentes, dans laquelle les granulés de prasugrel, ou d'un sel pharmaceutiquement acceptable de celui-ci, comprennent en outre au moins une couche d'enrobage qui contient du pullulane.

4. Formulation de comprimé pharmaceutique solide selon l'une quelconque des revendications précédentes, comprenant en outre au moins un, ou un mélange, de liants, de lubrifiants et d'agents de glissement.

5. Formulation de comprimé pharmaceutique solide selon l'une quelconque des revendications précédentes, dans laquelle ledit liant est sélectionné dans le groupe constitué par la polyvinylpyrrolidone (povidone), l'hydroxypropylméthylcellulose, l'hydroxypropylcellulose, la carboxyméthylcellulose, la méthylcellulose, l'éthylcellulose et la gélatine.

6. Formulation de comprimé pharmaceutique solide selon l'une quelconque des revendications précédentes, dans laquelle lesdit agent de glissement est sélectionnés dans le groupe constitué par le dioxide de silicium colloïdale, le talc, le silicate d'aluminium et le silicate de magnésium.

7. Formulation de comprimé pharmaceutique solide selon l'une quelconque des revendications précédentes, dans laquelle un désintégrant approprié est sélectionné dans le groupe constitué par le glycolate d'amidon sodique, la croscarmellose sodique, la crospovidone, l'alginate de sodium, les gommes, l'amidon et l'aluminosilicate de magnésium.

8. Formulation de comprimé pharmaceutique solide selon l'une quelconque des revendications précédentes, dans laquelle ledit lubrifiant est sélectionné dans le groupe constitué par le stéarylfumarate de sodium, le stéarate de magnésium, le polyéthylène glycol, l'acide stéarique, les stéarates de métaux, l'acide borique, le laurylsulfate de sodium ou de magnésium.

9. Formulation de comprimé pharmaceutique solide selon l'une quelconque des revendications précédentes, comprenant au moins une couche d'enrobage.

10. Formulation de comprimé pharmaceutique solide selon l'une quelconque des revendications précédentes, dans laquelle ladite couche d'enrobage comprend au moins un, ou un mélange, d'alcool polyvinylique, d'acide méthacrylique, d'hydroxypropylméthylcellulose et de carboxyméthylcellulose.

11. Formulation de comprimé pharmaceutique solide selon l'une quelconque des revendications précédentes, dans laquelle lesdits comprimés sont dans une plaquette thermoformée alu-alu.

12. Formulation de comprimé pharmaceutique solide selon l'une quelconque des revendications précédentes, constituée uniquement par les ingrédients suivants :
a. noyau
i. prasugrel, ou un sel pharmaceutiquement acceptable ou un polymorphe de celui-ci, à hauteur de 1 à 20 % en poids,
ii. pullulane à hauteur de 0,2 à 4 % en poids,
iii. cellulose microcristalline à hauteur de 5,0 à 90 % en poids,
iv. polyvinylpyrrolidone à hauteur de 0,25 à 5 % en poids,
v. croscarmellose sodique à hauteur de 0,2 à 10 % en poids,
vi. silice colloïdale à hauteur de 0,1 à 5 % en poids,
vii.stéarate de magnésium à hauteur de 0,1 à 10 % en poids,
b. enrobage
i. alcool polyvinylique à hauteur de 0,5 à 5 % en poids, ou
ii. hydroxypropylméthylcellulose à hauteur de 0,5 à 5 % en poids, ou un mélange adéquatement proportionné de celle-ci.

13. Formulation de comprimé pharmaceutique solide selon l'une quelconque des revendications précédentes, destinée à être utilisée dans la prévention ou le traitement de la thrombose, de l'embolie et des maladies cardiovasculaires chez les mammifères, et en particulier chez les humains.

14. Procédé de préparation d'une formulation de comprimé pharmaceutique solide selon l'une quelconque des revendications précédentes, comprenant les étapes de :
a. enrober les particules de l'agent actif avec du pullulane dans un système à lit fluidisé,
b. mélanger les particules d'agent actif enrobées de pullulane avec un agent de remplissage, un liant, un désintégrant et des lubrifiants-agents de glissement,
c. agglomérer par compression le mélange pour obtenir des comprimés, et
d. pelliculer les comprimés agglomérés par compression.
